# EUROPÄISCHE PATENTSCHRIFT

(11) **EP 3 280 972 B1**
(45) Veröffentlichungstag und Bekanntmachung des Hinweises auf die Patenterteilung: **13.03.2019**
(21) Anmeldenummer: 16721085.5
(22) Anmeldetag: 11.04.2016
(51) Int. Cl.: G01B 11/24, A61C 9/00, A61B 5/00

(54) **VERMESSUNGSSYSTEM UND VERFAHREN ZUR OPTISCHEN VERMESSUNG EINES OBJEKTS**
MEASUREMENT SYSTEM AND METHOD FOR OPTICALLY MEASURING AN OBJECT
SYSTÈME DE MESURE ET PROCÉDÉ DESTINÉS À LA MESURE OPTIQUE D'UN OBJET

(30) Priorität: 09.04.2015 DE 102015206341
(43) Veröffentlichungstag der Anmeldung: 14.02.2018
(73) Patentinhaber: Sirona Dental Systems GmbH, 64625 Bensheim (DE)
(72) Erfinder: ADAMSON, Anders, 64297 Darmstadt (DE); THIEL, Frank, 64372 Ober-Ramstadt (DE); WILLERS, Ulf, 64342 Seeheim-Jugenheim (DE)
(74) Vertreter: Sommer, Peter
(86) Internationale Anmeldenummer: PCT/EP2016/057870
(87) Internationale Veröffentlichungsnummer: WO 2016/162552

(56) Entgegenhaltungen:
- EP-A1- 0 438 353
- EP-A2- 1 238 624
- DE-A1- 10 043 749
- DE-B3-102013 223 894

## Beschreibung

### Technisches Gebiet

Die Erfindung betrifft ein Verfahren und ein Vermessungssystem zur optischen Vermessung eines Objekts, umfassend eine dentale Kamera und einen optischen Aufsatz.

### Stand der Technik

Aus dem Stand der Technik sind mehrere Verfahren und Vermessungsvorrichtungen zur optischen Vermessung eines dentalen Objekts bekannt.

DE 102013223894 B3 offenbart ein Vermessungssystem zur optischen Vermessung eines dentalen Objekts, umfassend eine dentale Kamera, die eine Lichtquelle, eine Aufnahmeeinheit und optische Komponenten zur Projektion eines Punktmusters auf das zu untersuchende Objekt und zur Weiterleitung der reflektierten Lichtstrahlen auf die Aufnahmeeinheit aufweist.

EP 0438353 A1 offenbart eine intraorale Kamera und ein Laser aufweisendes Instrument, wobei eine Fotolinse vor der Aufnahmeeinheit zur Aufnahme des ganzen Mundes angeordnet wird.

DE 100 43 749 A1 offenbart ein zahnmedizinisches Handstück mit Mitteln zur Bildaufnahme sowie Mittel, um eine Anregungsstrahlung auf einen zu untersuchenden Zahngewebebereich zu richten, wobei die Kamera zweiteilig aus einem Sondenkopf und einem Kameramodul aufgebaut ist. Durch Verwendung verschiedener Objekthülsen mit unterschiedlichen optischen Eigenschaften kann die Kamera mit unterschiedlichen Abbildungseigenschaften ausgestattet und somit an das jeweilige Anwendungsgebiet angepasst werden. Zur Übertragung des Bildes innerhalb des Aufsatzes wird eine Umlegungsvorrichtung in Form eines Prismas verwendet.
Ein Nachteil dieser dentalen Kamera besteht darin, dass die dentale Kamera lediglich zur Vermessung der Zähne verwendet werden kann.
Die Aufgabe der vorliegenden Erfindung besteht daher darin, ein Vermessungssystem zur optischen Vermessung bereit zu stellen, das nicht nur Zähne sondern auch größere Objekte aufnehmen kann.

### Darstellung der Erfindung

Die Erfindung gemäß dem unabhängigen Anspruch 1 betrifft ein Vermessungssystem zur optischen Vermessung eines Objekts, umfassend eine dentale Kamera und einen optischen Aufsatz. Der optische Aufsatz umfasst dabei mindestens eine Linse, die so geformt und angeordnet ist, dass der optische Aufsatz eine negative Brennweite aufweist, so dass durch den optischen Aufsatz ein Messfeld oder ein Messvolumen der dentalen Kamera vergrößert wird. Die dentale Kamera kann eine beliebige handgehaltene dentale Kamera sein, die auf einem zweidimensionalen oder auch auf einem dreidimensionalen Vermessungsverfahren beruht. Das Objekt können die Zähne des Patienten und das Gesicht des Patienten sein. Der optische Aufsatz kann mit Befestigungsmitteln ausgestattet sein, um an einer herkömmlichen dentalen Kamera befestigt zu werden. Die Befestigungsmittel können beispielsweise den Befestigungsmitteln einer bereits bekannten Spiegelhülse entsprechen.
Die Befestigungsmittel können beispielsweise einen Rastmechanismus und eine Blattfeder umfassen, so dass der Aufsatz beim Aufsetzen auf die dentale Kamera einrastet und damit in seiner Position relativ zur dentalen Kamera fixiert wird.

Der optische Aufsatz kann also eine einzelne Linse oder auch ein Linsensystem aus mehreren optischen Linsen umfassen, die zueinander so angeordnet sind und so geformt sind, dass der optische Aufsatz eine negative Brennweite aufweist und das Messvolumen aufweitet. Der optische Aufsatz kann beispielsweise aus einer plankonvexen Linse und einer konvexplanen Linse oder aus einer konkav konvexen Linse und einer konvex konkaven Linse bestehen. Das Messfeld ist der Bereich innerhalb dessen die dentale Kamera das Objekt vermisst. Bei einer dreidimensionalen optischen dentalen Kamera wird ein Messvolumen des Objekts vermessen. Durch den optischen Aufsatz wird dieses Messfeld bei einer zweidimensionalen Kamera oder ein Messvolumen bei einer dreidimensionalen Kamera deutlich vergrößert, sodass größere Objekte vermessen werden können.

Ein Vorteil des Vermessungssystems besteht darin, dass eine herkömmliche dentale Kamera nicht nur zur Vermessung der Zähne, sondern durch den optischen Aufsatz auch zur Vermessung größerer Objekte, wie des Gesichtes eines Patienten oder ein Teilbereich des Gesichtes verwendet werden kann.

Vorteilhafterweise kann die dentale Kamera auf einem zweidimensionalen Vermessungsverfahren oder auf einem dreidimensionalen Vermessungsverfahren beruhen.

Dadurch kann der optische Aufsatz sowohl für zweidimensionale dentale Kameras als auch für dreidimensionale dentale Kameras verwendet werden.

Vorteilhafterweise kann die dentale Kamera auf einem zweidimensionalen Videoaufnahmeverfahren, einem dreidimensionalen Triangulationsmessverfahren, einem dreidimensionalen konfokalen Vermessungsverfahren oder einem Weißlichtinterferometrievermessungsverfahren beruhen.

Das dreidimensionale Triangulationsmessverfahren kann beispielsweise das bekannte Streifenprojektionsverfahren sein, bei dem ein Muster aus hellen und dunklen Streifen auf das Messprojekt projiziert wird. Das projizierte Streifenmuster wird dann unter einem bekannten Blickwinkel zur Projektion mittels der dentalen Kamera aufgenommen. Unter Verwendung eines sogenannten Phasenschiebeverfahrens wird eine Projektionskoordinate bestimmt, die die Position des Streifens im Muster wiedergibt. Bei einem bekannten Triangulationswinkel zwischen einem Beleuchtungsstrahl und einem Beobachtungsstrahl kann die 3D-Raumkoordinate des jeweiligen Messpunktes des Objekts ermittelt werden. Auf diese Weise wird für jeden Messpunkt des Objekts die Raumkoordinate ermittelt und ein dreidimensionales Bild der Oberfläche des Objekts berechnet.

Bei den dreidimensionalen konfokalen Vermessungsverfahren wird die Oberfläche des dentalen Objekts schrittweise abgetastet, wobei eine Fokalebene schrittweise verschoben wird. Das Licht außerhalb der Fokalebene wird mittels einer Lochblende möglichst ausgeblendet. Aus den gemessenen Bilddaten der einzelnen Schritte unterschiedlicher Fokalebenen kann anschließend ein dreidimensionales Bild des vermessenen Objekts berechnet werden.

Das zweidimensionale Videoaufnahmeverfahren ist ein bekanntes Vermessungsverfahren, bei dem eine Abfolge von zweidimensionalen Bildern des Objekts erzeugt wird.

Bei der Weißlichtinterferometrie wird ein Licht geringer Kohärenzlänge verwendet, so dass farbige Interferenzen entstehen, wenn die Weglänge im Referenz- oder Objektstrahl nahezu gleich sind. Beim Verändern der Weglänge wird das Interferenzmuster verändert, so dass anhand des Interferenzmusters der Abstand zur Oberfläche des Objekts bestimmt werden kann.

Die dentale Kamera kann auch auf einem Vermessungsverfahren beruhen, das eine Kombination aus einem zweidimensionalen Videoaufnahmeverfahren und einem dreidimensionalen Triangulationsverfahren darstellt, wobei die dreidimensionale Aufnahme aus dem Triangulationsmessverfahren mit der farbigen zweidimensionalen Videoaufnahme überlagert wird, so dass eine dreidimensionale farbige Aufnahme erzeugt wird.

Vorteilhafterweise kann der optische Aufsatz mit der dentalen Kamera trennbar mittels eines Verbindungsmittels verbunden sein.

Dadurch kann der Benutzer kleinere Objekte, wie Zähne, mittels der Kamera ohne Aufsatz und größere Objekte, wie Teilbereiche des Gesichtes, mittels der Kamera mit dem Aufsatz vermessen. Die Befestigungsmittel des optischen Aufsatzes an der Kamera sind so ausgestaltet, dass der optische Aufsatz in einer fest definierten Lage und Ausrichtung relativ zur Kamera angeordnet wird. Die Verbindungsmittel können beispielsweise eine Schraubverbindung sein.

Vorteilhafterweise kann mittels des optischen Aufsatzes das Messfeld oder das Messvolumen der dentalen Kamera um einen Vergrößerungsfaktor vergrößert sein, der mindestens 5 beträgt.

Dadurch kann also ein typisches Messvolumen einer dentalen Kamera in der Form eines Würfels mit einer Kantenlänge zwischen 15 mm und 20 mm auf ein vergrößertes Messvolumen aufgeweitet werden, das beispielsweise eine Kantenlänge von mindestens 30 mm aufweist. Das muss Volumen kann auch eine deutlich größere Kantenlänge, wie beispielsweise von mindestens 100 mm aufweisen. Die Kantenlänge des Messvolumens sollte so gewählt werden, dass relevante Gesichtspartien, wie der Mund, die Nase und/oder die Augen registriert werden können.
Vorteilhafterweise kann der optische Aufsatz zusätzlich einen Strahlumlenker umfassen, der einen Beleuchtungsstrahl und einen Beobachtungsstrahl der dentalen Kamera zum Objekt umlenkt.
Der Strahlumlenker kann beispielsweise ein Spiegel oder ein Prisma sein, wobei auf diese Weise die Vermessungsrichtung der Aufnahme senkrecht zur Längsrichtung der Kamera verlaufen kann. Insbesondere bei der Vermessung der Zähne ist dies vorteilhaft.
Vorteilhafterweise kann der optische Aufsatz mehrere Linsen umfassen, die so geformt sind und zueinander so angeordnet sind, dass der optische Aufsatz eine negative Brennweite aufweist.
Dadurch werden also mehrere optische Linsen verwendet, um die negative Brennweite zu erzeugen.
Vorteilhafterweise kann mittels des optischen Aufsatzes das Messvolumen der dreidimensionalen dentalen Kamera in Form eines Würfels mit einer Kantenlänge zwischen 10 mm und 20 mm auf ein vergrößertes Messvolumen mit einer Kantenlänge von mindestens 30 mm vergrößert werden.
Dadurch kann mittels der Kamera mit dem Aufsatz ein Teilbereich des Gesichtes vermessen werden.
Die Erfindung gemäß dem unabhängigen Anspruch 9 betrifft weiterhin ein Verfahren zur Vermessung eines Objekts mittels einer dentalen Kamera mit einem ) abtrennbaren optischen Aufsatz. Der optische Aufsatz weist dabei eine negative Brennweite auf, so dass durch den optischen Aufsatz ein Messfeld oder ein Messvolumen der dentalen Kamera vergrößert wird.

Das vorliegende Verfahren ermöglicht also mittels einer herkömmlichen dentalen Kamera mit dem neuartigen optischen Aufsatz ein größeres Messfeld zu vermessen.

Vorteilhafterweise kann die dentale Kamera auf einem zweidimensionalen Vermessungsverfahren oder auf einem dreidimensionalen Vermessungsverfahren beruhen.

Dadurch kann das vorliegende Verfahren auch auf eine zweidimensionale oder eine dreidimensionale dentale Kamera angewendet werden.

Vorteilhafterweise kann die dentale Kamera auf einem zweidimensionalen Videoaufnahmeverfahren, einem dreidimensionalen Triangulationsmessverfahren, einem dreidimensionalen konfokalen Vermessungsverfahren oder einem Weißlichtinterferometrievermessungsverfahren beruhen.

Dadurch kann das Verfahren für eine dentale Kamera verwendet werden, die auf den genannten Vermessungsverfahren beruht.

Vorteilhafterweise kann mittels des optischen Aufsatzes das Messfeld oder das Messvolumen der dentalen Kamera um einen Vergrößerungsfaktor vergrößert werden, der mindestens 5 beträgt.

Dadurch wird also das Messfeld deutlich vergrößert.

Vorteilhafterweise können in einem ersten Schritt mittels der dentalen Kamera ohne den optischen Aufsatz die Zähne eines Patienten vermessen werden und eine erste Aufnahme der Zähne erzeugt wird, wobei in einem zweiten Schritt mittels der dentalen Kamera mit dem optischen Aufsatz mit dem vergrößerten Messfeld bzw. Messvolumen zumindest ein Teil eines Gesichtes des Patienten vermessen wird und eine zweite Aufnahme des Gesichtes erzeugt wird, wobei anschließend die erste Aufnahme der Zähne und die zweite Aufnahme des Gesichtes zu einer Gesamtaufnahme registriert werden.

Dadurch kann mittels der dentalen Kamera ohne Aufsatz die Zahnsituation und mittels der Kamera mit dem Aufsatz zumindest ein Teilbereich des Gesichtes vermessen werden. Der vermessene Teilbereich des Gesichtes kann dabei beispielsweise charakteristische Bereiche, wie Nasenflügel, den Verlauf des Kinns, sowie gleichzeitig auch die Zähne, insbesondere die Höckerspitzen der Eckzähne umfassen. Die aufgenommenen Zähne können durch die präzisere Aufnahme der Zähne mittels der Kamera ohne Aufsatz bei der Registrierung ersetzt werden. Eine solche Aufnahme die sowohl einen Teil des Gesichtes als auch die Zähne umfasst, ermöglicht dem Zahntechniker oder dem Zahnarzt eine Therapieplanung unter Berücksichtigung der Gesichtsform, der Nasenflügelbreite oder dem Verlauf des Kinns. Bei der Planung von Zahnersatzteilen können also diese Informationen verwendet werden. Beispielsweise können die Zahnersatzteile so geplant werden, dass der Abstand zwischen den Höckerspitzen der beiden Eckzähne mit der Nasenflügelbreite übereinstimmt, um einen ästhetischen optischen Eindruck von natürlichen Zähnen nachzuahmen.

### Kurzbeschreibung der Zeichnungen

Die Erfindung wird anhand der Zeichnungen erläutert. Es zeigt, die
- Fig. 1: ein Vermessungssystem umfassend eine dentale Kamera und einen optischen Aufsatz; die
- Fig. 2: eine Skizze zur Verdeutlichung der Strahlengänge der Beobachtungsstrahlen der dentalen Kamera ohne den optischen Aufsatz; die
- Fig. 3: eine Skizze zur Verdeutlichung der Strahlengänge der Beobachtungsstrahlen der dentalen Kamera mit dem optischen Aufsatz; die
- Fig. 4: zeigt eine Aufnahme eines Teilbereichs des Gesichtes und der Zähne mittels der Kamera mit dem optischen Aufsatz.

### Ausführungsbeispiele

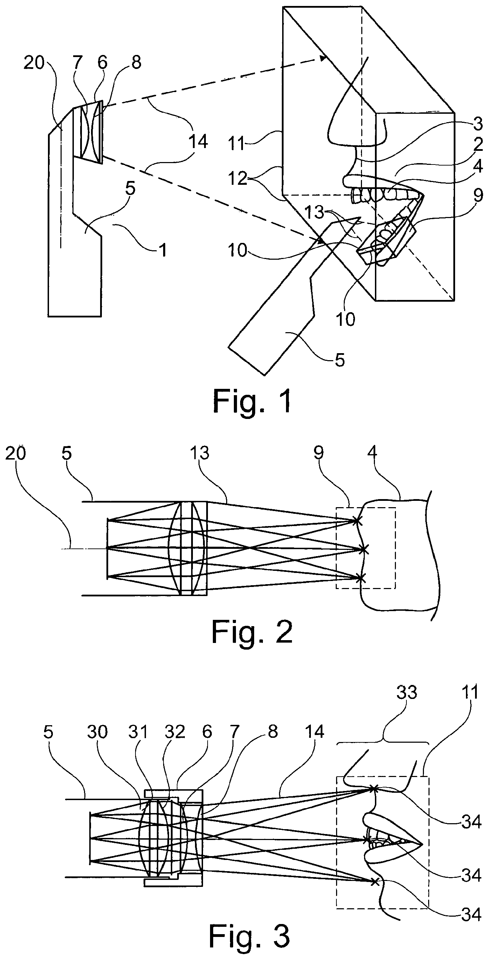

Die Fig. 1 zeigt ein Vermessungssystem 1 zur optischen Vermessung eines Objekts 2, das ein Gesicht des Patienten 3 und/oder die Zähne 4 eines Patienten sein können. Das Vermessungssystem 1 umfasst eine herkömmliche dentale Kamera 5 und einen optischen Aufsatz 6, der eine erste plankonvexe Linse 7 und eine zweite konvexplane Linse 8 aufweist. Die beiden Linsen 7 und 8 sind so geformt und relativ zur Kamera 5 so angeordnet, dass der optische Aufsatz 6 eine negative Brennweite aufweist. Mittels der Kamera 5 ohne den optischen Aufsatz 6 werden kleine Objekte, wie die Zähne 4 vermessen, wobei ein Messvolumen 9 der dentalen Kamera 5 relativ klein ist und bei einer würfelförmigen Form beispielsweise eine Kantenlänge 10 zwischen 10 mm und 20 mm aufweisen kann. Durch den optischen Aufsatz 6 wird dieses Messvolumen 9 auf ein vergrößertes Messvolumen 11 mit einer Kantenlänge 12 von mindestens 100 mm vergrößert. Die ersten Beleuchtungsstrahlen 13 der dentalen Kamera 5 ohne den optischen Aufsatz 6 sind durch die gestrichelten Linien angedeutet. Die zweiten Beleuchtungsstrahlen 14 der dentalen Kamera 5 mit dem optischen Aufsatz 6 definieren die Grenzen des vergrößerten Messvolumens 11.

Das Vermessungssystem 1 kann also für ein Verfahren verwendet werden, bei dem im ersten Schritt mittels der Kamera 5 ohne den optischen Aufsatz 6 die Zähne 4 vermessen werden und anschließend im zweiten Schritt mittels der derselben Kamera 5 mit dem optischen Aufsatz 6 ein Teilbereich des Gesichtes 3 des Patienten vermessen wird. Die beiden Aufnahmen können dann anschließend zueinander registriert werden. Die dentale Kamera 5 kann auf einem dreidimensionalen Triangulationsverfahren oder auf einem konfokalen Vermessungsverfahren beruhen.

Die Fig. 2 zeigt eine Skizze zur Verdeutlichung der Strahlengänge der Beleuchtungsstrahlen 14 der dentalen Kamera 5 ohne den optischen Aufsatz 6. Die Bauform der dentalen Kamera 5 kann beliebig gestaltet sein. Dabei kann die dentale Kamera 5 eine Spiegelhülse aufweisen, die in Fig. 1 dargestellt ist, die die Beleuchtungsstrahlen 14 senkrecht zur Längsachse der dentalen Kamera 5 umlenkt. Die dentale Kamera 5 kann jedoch auch ohne Spiegelhülse verwendet werden, wie in Fig. 2 dargestellt ist. Dabei werden die Beleuchtungsstrahlen 14 in die Richtung einer Längsachse 20 der dentalen Kamera 5 abgestrahlt. In Fig. 2 ist ein kleines Messvolumen 9 durch die gestrichelte Linie dargestellt, das lediglich einen Teilbereich eines Zahns vermisst.

Die Fig. 3 zeigt eine Skizze der Kamera 5 aus Fig. 2 mit dem optischen Aufsatz 6 umfassend eine erste Linse 7 und eine zweite Linse 8. Der optische Aufsatz 6 wird also im Strahlengang der Beleuchtungsstrahlen 14 des aufgeweiteten Messfeldes 11 zusätzlich zum Objektiv 30 der dentalen Kamera 5 bestehend aus einer ersten Linse 31 und einer zweiten Linse 32 angeordnet. Dadurch wird also das Messvolumen 9 der dentalen Kamera 5 bis zum aufgeweiteten Messvolumen 11 vergrößert, wobei nicht nur die Kantenlänge 10 sondern auch ein Tiefenmessbereich 33 vergrößert wird. Bei dem Triangulationsmessverfahren beruht die Vergrößerung des Tiefenmessbereichs darauf, dass der Triangulationswinkel aufgrund des längeren Fokus entsprechend verkleinert wird. Beim konfokalen Vermessungsverfahren führt der längere Fokus ebenfalls zur Vergrößerung des Tiefenmessbereichs. In Fig. 3 ist schematisch dargestellt, wie die Beleuchtungsstrahlen 14 aufgeweitet werden und dadurch auch die Fokuspunkte 34 weiter auseinander liegen, als in Fig. 1.

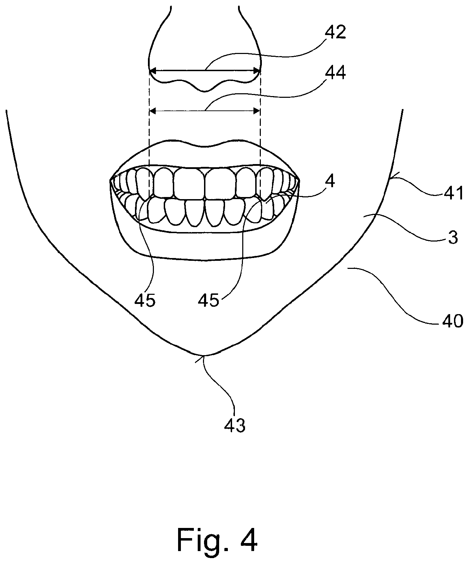

Die Fig. 4 zeigt eine Aufnahme 40 eines Teilbereichs des Gesichtes 3 und der Zähne 4 mittels der Kamera 5 mit dem optischen Aufsatz 6 aus Fig. 1. Zur Verbesserung der Genauigkeit kann die präzisere Aufnahme der Zähne mittels der Kamera 5 ohne Aufsatz 6 mit der Aufnahme des Gesichtes 3 registriert werden. Denn die Aufnahme der Zähne 4 mittels der Kamera 5 ohne Aufsatz 6 weist eine höhere Auflösung auf. Die Aufnahme 40 des Gesichtes 3 ermöglicht es dem Zahntechniker oder dem Zahnarzt eine Therapieplanung unter Berücksichtigung einer Gesichtsform 41, einer Nasenflügelbreite 42, einem Verlauf des Kinns 43 und/ oder einem Abstand 44 von Höckerspitzen 45 der Eckzähne durchzuführen. Im dargestellten Fall aus Fig. 4 stimmt die Nasenflügelbreite 42 mit dem Abstand 44 überein. Dies entspricht einer natürlichen Gegebenheit bei den meisten Menschen. Sei der Planung von Zahnrestaurationen wird also auch die Nasenflügelbreite 42 berücksichtigt, um natürliche Zähne nachzuahmen.

### Bezugszeichen

- 1: Vermessungssystem
- 2: Objekt
- 3: Patient / Gesicht des Patienten
- 4: Zähne
- 5: dentale Kamera
- 6: optischer Aufsatz
- 7: erste Linse
- 8: zweite Linse
- 9: Messvolumen
- 10: Kantenlänge
- 11: vergrößertes Messvolumen
- 12: Kantenlänge
- 13: erste Beleuchtungsstrahlen
- 14: zweite Beobachtungsstrahlen
- 20: Längsachse
- 30: Objektiv
- 31: erste Linse
- 32: zweite Linse
- 33: Tiefenmessbereich
- 34: Fokuspunkte
- 40: Aufnahme
- 41: Gesichtsform
- 42: Nasenflügelbreite
- 43: Kinn
- 44: Abstand
- 45: Höckerspitzen

## Patentansprüche

1. Vermessungssystem (1) zur optischen Vermessung eines Objekts (2), umfassend eine dentale Kamera (5) und einen optischen Aufsatz (6), **dadurch gekennzeichnet, dass** der optische Aufsatz (6) mindestens eine Linse (7, 8) umfasst, die so geformt und angeordnet ist, dass der optische Aufsatz (6) eine negative Brennweite aufweist, so dass durch den optischen Aufsatz (6) ein Messfeld oder ein Messvolumen (9) der dentalen Kamera (5) vergrößert wird.

2. Vermessungssystem nach Anspruch 1, **dadurch gekennzeichnet, dass** die dentale Kamera (5) auf einem zweidimensionalen Vermessungsverfahren oder auf einem dreidimensionalen Vermessungsverfahren beruht.

3. Vermessungssystem nach Anspruch 2, **dadurch gekennzeichnet, dass** die dentale Kamera (5) auf einem zweidimensionalen Videoaufnahmeverfahren, einem dreidimensionalen Triangulationsmessverfahren, einem dreidimensionalen konfokalen Vermessungsverfahren oder einem Weißlichtinterferometrievermessungsverfahren beruht.

4. Vermessungssystem nach einem der Ansprüche 1 bis 3, **dadurch gekennzeichnet, dass** der optische Aufsatz (6) mit der dentalen Kamera (5) trennbar mittels eines Verbindungsmittels verbunden ist.

5. Vermessungssystem nach einem der Ansprüche 1 bis 4, **dadurch gekennzeichnet, dass** mittels des optischen Aufsatzes (6) das Messfeld oder das Messvolumen (9) der dentalen Kamera (5) um einen Vergrößerungsfaktor vergrößert wird, der mindestens 5 beträgt.

6. Vermessungssystem nach einem der Ansprüche 1 bis 5, **dadurch gekennzeichnet, dass** der optische Aufsatz (6) zusätzlich einen Strahlumlenker umfasst, der einen Beleuchtungsstrahl (13, 14) und einen Beobachtungsstrahl der dentalen Kamera (5) zum Objekt (3, 4) umlenkt.

7. Vermessungssystem nach einem der Ansprüche 1 bis 6, **dadurch gekennzeichnet, dass** der optische Aufsatz (6) mehrere Linsen (7, 8) umfasst, die so geformt sind und zueinander so angeordnet sind, dass der optische Aufsatz (6) eine negative Brennweite aufweist.

8. Vermessungssystem nach einem der Ansprüche 1 bis 7, **dadurch gekennzeichnet, dass** mittels des optischen Aufsatzes (6) das Messvolumen (9) der dreidimensionalen dentalen Kamera (5) in Form eines Würfels mit einer Kantenlänge zwischen 10 mm und 20 mm auf ein vergrößertes Messvolumen (11) mit einer Kantenlänge von mindestens 30 mm vergrößert wird.

9. Verfahren zur Vermessung eines Objekts (3, 4) mittels einer dentalen Kamera (5) mit einem abtrennbaren optischen Aufsatz (6), **dadurch gekennzeichnet, dass** der optische Aufsatz (6) eine negative Brennweite aufweist, so dass durch den optischen Aufsatz (6) ein Messfeld oder ein Messvolumen (9) der dentalen Kamera vergrößert wird.

10. Verfahren nach Anspruch 9, **dadurch gekennzeichnet, dass** die dentale Kamera (5) auf einem zweidimensionalen Vermessungsverfahren oder auf einem dreidimensionalen Vermessungsverfahren beruht.

11. Verfahren nach Anspruch 10, **dadurch gekennzeichnet, dass** die dentale Kamera (5) auf einem zweidimensionalen Videoaufnahmeverfahren, einem dreidimensionalen Triangulationsmessverfahren, einem dreidimensionalen konfokalen Vermessungsverfahren oder einem Weißlichtinterferometrievermessungsverfahren beruht.

12. Verfahren nach einem der Ansprüche 9 bis 11, **dadurch gekennzeichnet, dass** mittels des optischen Aufsatzes (6) das Messfeld oder das Messvolumen (9) der dentalen Kamera (5) um einen Vergrößerungsfaktor vergrößert wird, der mindestens 5 beträgt.

13. Verfahren nach einem der Ansprüche 9 bis 12, **dadurch gekennzeichnet, dass** mittels der dentalen Kamera (5) ohne den optischen Aufsatz (6) die Zähne (4) eines Patienten vermessen werden und eine erste Aufnahme der Zähne erzeugt wird, wobei mittels der dentalen Kamera (5) mit dem optischen Aufsatz (6) mit dem vergrößerten Messfeld bzw. Messvolumen (11) zumindest ein Teil eines Gesichtes (3) des Patienten vermessen wird und eine zweite Aufnahme des Gesichtes erzeugt wird, wobei anschließend die erste Aufnahme der Zähne (4) und die zweite Aufnahme des Gesichtes (3) zu einer Gesamtaufnahme registriert werden.

## Claims

1. Measuring system (1) for optically measuring an object (2), comprising a dental camera (5) and an optical attachment (6), **characterized in that** the optical attachment (6) comprises at least one lens (7, 8) which is shaped and disposed such that the optical attachment (6) has a negative focal length, so that a measurement field or a measurement volume (9) of the dental camera (5) is enlarged by the optical attachment (6).

2. Measuring system according to Claim 1, **characterized in that** the dental camera (5) is based on a two-dimensional measuring method or on a three-dimensional measuring method.

3. Measuring system according to Claim 2, **characterized in that** the dental camera (5) is based on a two-dimensional video recording method, a three-dimensional triangulation measuring method, a three-dimensional confocal measuring method or a white light interferometry measuring method.

4. Measuring system according to any one of Claims 1 to 3, **characterized in that** the optical attachment (6) is detachably connected to the dental camera (5) by means of a connecting means.

5. Measuring system according to any one of Claims 1 to 4, **characterized in that**, by means of the optical attachment (6), the measurement field or the measurement volume (9) of the dental camera (5) is increased by an enlargement factor of at least 5.

6. Measuring system according to any one of Claims 1 to 5, **characterized in that** the optical attachment (6) additionally comprises a beam deflector, which deflects an illumination beam (13, 14) and an observation beam of the dental camera (5) toward the object (3, 4).

7. Measuring system according to any one of Claims 1 to 6, **characterized in that** the optical attachment (6) comprises a plurality of lenses (7, 8), which are shaped and disposed relative to one another such that the optical attachment (6) has a negative focal length.

8. Measuring system according to any one of Claims 1 to 7, **characterized in that**, by means of the optical attachment (6), the measurement volume (9) of the three-dimensional dental camera (5) in the shape of a cube with an edge length between 10 mm and 20 mm is enlarged to an enlarged measurement volume (11) with an edge length of at least 30 mm.

9. Method for measuring an object (3, 4) by means of a dental camera (5) having a detachable optical attachment (6), **characterized in that** the optical attachment (6) has a negative focal length, so that a measurement field or a measurement volume (9) of the dental camera is enlarged by means of the optical attachment (6).

10. Method according to Claim 9, **characterized in that** the dental camera (5) is based on a two-dimensional measuring method or on a three-dimensional measuring method.

11. Method according to Claim 10, **characterized in that** the dental camera (5) is based on a two-dimensional video recording method, a three-dimensional triangulation measuring method, a three-dimensional confocal measuring method or a white light interferometry measuring method.

12. Method according to any one of Claims 9 to 11, **characterized in that**, by means of the optical attachment (6), the measurement field or the measurement volume (9) of the dental camera (5) is increased by an enlargement factor of at least 5.

13. Method according to any one of Claims 9 to 12, **characterized in that** the teeth (4) of a patient are measured and a first recording of the teeth is produced by means of the dental camera (5) without the optical attachment (6), wherein at least part of the face (3) of the patient is measured and a second recording of the face is produced by means of the dental camera (5) with the optical attachment (6) with the enlarged measurement field or measurement volume (11), wherein the first recording of the teeth (4) and the second recording of the face (3) are subsequently registered to create one composite recording.

## Revendications

1. Système de mesure (1) destiné à la mesure optique d'un objet (2), comprenant une caméra dentaire (5) et un élément optique rapporté (6), **caractérisé en ce que** l'élément optique rapporté (6) comprend au moins une lentille (7, 8) qui est formée et disposée de manière que l'élément optique rapporté (6) présente une distance focale négative, ce qui permet à l'élément optique rapporté (6) d'agrandir un champ de mesure ou un volume de mesure (9) de la caméra dentaire (5).

2. Système de mesure selon la revendication 1, **caractérisé en ce que** la caméra dentaire (5) s'appuie sur un procédé de mesure bidimensionnelle ou sur un procédé de mesure tridimensionnelle.

3. Système de mesure selon la revendication 2, **caractérisé en ce que** la caméra dentaire (5) s'appuie sur un procédé de prise de vues vidéo bidimensionnelles, sur un procédé de triangulation tridimensionnelle, sur un procédé de mesure confocale tridimensionnelle ou sur un procédé de mesure par interférométrie en lumière blanche.

4. Système de mesure selon une des revendications 1 à 3, **caractérisé en ce que** l'élément optique rapporté (6) est raccordé à la caméra dentaire (5) de manière séparable par le biais d'un moyen de raccordement.

5. Système de mesure selon une des revendications 1 à 4, **caractérisé en ce que** l'élément optique rapporté (6) permet d'agrandir le champ de mesure ou le volume de mesure (9) de la caméra dentaire (5) selon un facteur d'agrandissement d'au moins 5.

6. Système de mesure selon une des revendications 1 à 5, **caractérisé en ce que** l'élément optique rapporté (6) comprend en outre un dispositif de déviation de faisceau qui dévie un faisceau d'éclairage (13, 14) et un faisceau d'observation de la caméra dentaire (5) vers l'objet (3, 4).

7. Système de mesure selon une des revendications 1 à 6, **caractérisé en ce que** l'élément optique rapporté (6) comprend plusieurs lentilles (7, 8) qui sont formées et sont disposées les unes par rapport aux autres de telle manière que l'élément optique rapporté (6) présente une distance focale négative.

8. Système de mesure selon une des revendications 1 à 7, **caractérisé en ce que** l'élément optique rapporté (6) permet d'agrandir le volume de mesure (9) de la caméra dentaire tridimensionnelle (5) selon une forme de cube d'une longueur d'arête comprise entre 10 mm et 20 mm en un volume de mesure agrandi (11) d'une longueur d'arête d'au moins 30 mm.

9. Procédé de mesure d'un objet (3, 4) au moyen d'une caméra dentaire (5) dotée d'un élément optique rapporté (6) séparable, **caractérisé en ce que** l'élément optique rapporté (6) présente une distance focale négative, ce qui permet à l'élément optique rapporté (6) d'agrandir un champ de mesure ou un volume de mesure (9) de la caméra dentaire.

10. Procédé selon la revendication 9, **caractérisé en ce que** la caméra dentaire (5) s'appuie sur un procédé de mesure bidimensionnelle ou sur un procédé de mesure tridimensionnelle.

11. Procédé selon la revendication 10, **caractérisé en ce que** la caméra dentaire (5) s'appuie sur un procédé de prises de vues vidéo bidimensionnelles, un procédé de triangulation tridimensionnelle, un procédé de mesure confocale tridimensionnelle ou un procédé de mesure par interférométrie en lumière blanche.

12. Procédé selon une des revendications 9 à 11, **caractérisé en ce que** l'élément optique rapporté (6) permet d'agrandir le champ de mesure ou le volume de mesure (9) de la caméra dentaire (5) selon un facteur d'agrandissement d'au moins 5.

13. Procédé selon une des revendications 9 à 12, **caractérisé en ce que** la caméra dentaire (5) sans l'élément optique rapporté (6) permet de prendre des mesures des dents (4) d'un patient et de produire une première prise de vue des dents, ladite caméra dentaire (5) dotée de l'élément optique rapporté (6), offrant ledit champ de mesure ou volume de mesure (11) agrandi, permettant de prendre les mesures d'au moins une partie du visage (3) du patient et de produire une deuxième prise de vue du visage, ladite première prise de vue des dents (4) et ladite deuxième prise de vue du visage (3) étant ensuite inscrites dans un enregistrement global.
